# EUROPEAN PATENT SPECIFICATION

(11) **EP 3 746 142 B1**
(45) Date of publication and mention of the grant of the patent: **24.12.2025**
(21) Application number: 19705556.9
(22) Date of filing: 11.01.2019
(51) Int. Cl.: A61M 1/00, A47L 9/02

(54) **DEVICE FOR REMOVING BY SUCTION A BODILY FLUID, PRIMARILY NASAL FLUID**
VORRICHTUNG ZUM ABSAUGEN EINER KÖRPERFLÜSSIGKEIT, HAUPTSÄCHLICH NASALER FLÜSSIGKEIT
DISPOSITIF D'ÉLIMINATION PAR ASPIRATION DE FLUIDE CORPOREL, PRINCIPALEMENT DE FLUIDE NASAL

(30) Priority: 31.01.2018 HU 1800039
(43) Date of publication of application: 09.12.2020
(73) Proprietor: Illés Csók És Társa KFT, 1146 Budapest (HU)
(72) Inventor: ILLÉS CSÓK, Anna, 1146 Budapest (HU)
(74) Representative: Szentpéteri, Zsolt
(86) International application number: PCT/HU2019/000001
(87) International publication number: WO 2019/150143

(56) References cited:
- EP-A1- 0 752 893
- WO-A2-99/53819
- DE-U1- 9 420 699
- FR-A1- 2 728 469
- FR-A1- 2 912 062
- HU-U- 3 471
- HU-U- 3 472
- RU-U1- 87 082
- RU-U1- 87 083
- US-B2- 9 649 414

## Description

### Technical field of the invention

The disclosure relates to a device for removing a bodily fluid, particularly nasal fluid applying a vacuum source, preferably a vacuum cleaner, comprising a body adapted for receiving a collector vessel fitted with a suction inlet opening and an outlet opening, and an interconnection pipe fitted with a connection member connecting the outlet opening to the vacuum source.

### Background art

The prior art includes a number of different devices for removing unwanted exudates from the human body. An example of known devices for removing, by suction, nasal fluids is a pipette terminated by a rubber cap, wherein the fluid is removed applying vacuum produced by manually compressing the rubber cap. A common drawback of these devices is that they are not capable of completely removing the exudate, and at the same time are either not suited for reuse or reuse involves the risk of infection.

To address this problem, Hungarian patent HU 215 563 discloses a device that is adapted for being connected to a vacuum cleaner and has a collector vessel for receiving the removed bodily fluid.

A number of technical solutions addressing the problems related to the use of the device are included in the prior art.

Such technical solutions are disclosed in the utility models No. HU 3471 and HU 3472, wherein the suction head of the device is connected to the collector vessel, and the device itself consists of releasably interconnected bottom and upper portions that have conical and arcuate configuration.

A common disadvantage of known devices is that the collector vessel of the device is in many cases incapable of retaining the removed bodily fluid, resulting in that the exudate enters the connection pipe of the vacuum cleaner (or the vacuum cleaner itself) due the suction effect resulting from the applied vacuum, causing undesirable contamination.

This is dependent on the consistency of the bodily fluid being removed.

In recent years, pharmaceutical companies have introduced a number of allegedly antibacterial/medicinal nasal sprays that are capable of loosing and diluting deposited exudates.

After dilution, the bodily exudate has a water-like consistency, and therefore it cannot be safely removed applying known devices because the vacuum-generated airflow can carry it off from the collector vessel. Another prior art device is disclosed in WO99/53819.

### Disclosure of the invention

The objective of the invention is to provide a device that, while preserving the advantageous characteristics of known technical solutions, is capable of safely removing and storing diluted, water-like bodily fluids.

The objective of the invention has been fulfilled by providing a device as defined in claim 1.

In another preferred embodiment of the device according to the invention, the suction bell comprises protrusions at its bottom portion connected to the discharge bell, and the discharge bell comprises seats that are disposed at the upper portion thereof and are adapted for receiving the protrusion of the suction bell.

In all preferred embodiments of the device according to the invention the suction bell and the discharge bell have a slightly conical configuration, and the suction bell, the discharge bell, their subcomponents, and the central member, as well as the connector members - such as the torch-shaped member fitted with a suction cone - are made of plastic.

### Brief description of the drawings

A preferred embodiment of the device according to the invention is explained in detail referring to the accompanying drawings, where
Fig. 1 is a sectional view of the device according to the invention,
Fig. 2 is the sectional view of the central member of the device shown in Fig. 1, and
Fig. 3 is the top plan view of the central member illustrated in Fig. 2.

### Detailed description of the invention

The device 1 according to the invention is adapted for removing by suction a bodily fluid, primarily nasal fluid, and can be preferably attached to a vacuum cleaner suction pipe.

The device 1 consists of a releasably interconnected suction bell 2 and discharge bell 3, with a central member 4 being included between the suction bell 2 and the discharge bell 3.

The suction bell 2 is adapted to receive a torch-shaped member 5 having a replaceable suction cone 6, with the bottom portion of the torch-shaped member 5 extending into the receiving member 14 of the central member 4.

A connection pipe 7 that is coaxial with the torch-shaped member 5 situated in the suction pipe 2 is disposed at the bottom portion of the discharge bell 3, with the upper portion 8 thereof extending towards the central member 4 inside the discharge bell 3, and with the bottom portion 9 thereof extending over the end of the discharge bell 3 and forming a connection stub adapted for receiving a connection member.

The bottom portion of the discharge bell 3 is fitted with a protrusion 10 parallel with the bottom portion 9 of the connection pipe 7 such that, together with the bottom portion 9 of the connection pipe 7, it forms a seat adapted for receiving a connection member not shown in the drawings.

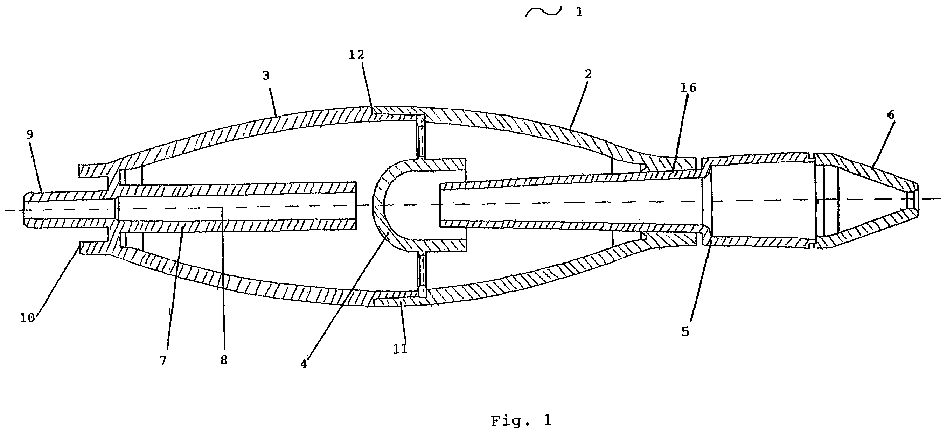

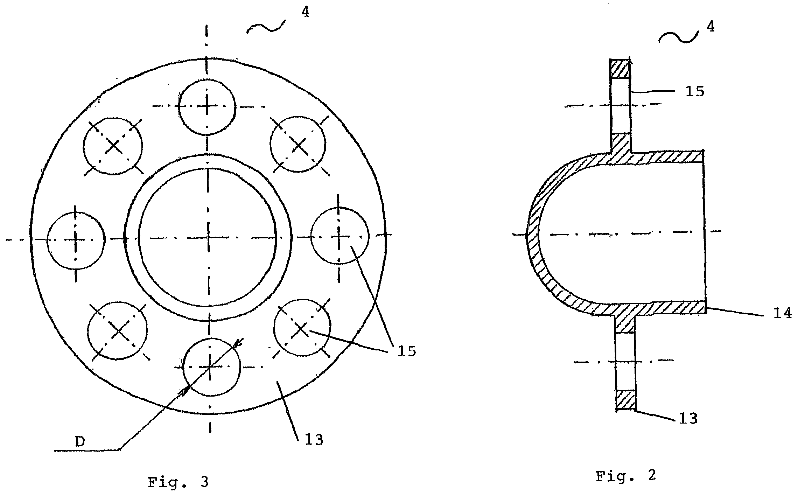

Fig. 2 illustrates in sectional view the central member 4 of the device 1 according to Fig. 1 that is essentially formed of a receiving space 14 and a disc 13 adjoining the receiving space 14 and being made integral therewith, the disc 13 comprising bores 15 having a diameter D (see Fig. 3).

The device according to the invention is operated as follows.

In a state wherein the device 1 is ready for use, the discharge bell 3 and the suction bell 2 are snapped together. To provide a safe connection, the protrusion 11 of the suction bell 2 is passed into a seat 12 formed on the upper portion of the discharge bell 3, and then the suction bell 2 and the discharge bell 3 are interconnected by slightly rotating the suction bell 2 along the discharge bell 3.

It should be noted that the protrusion 11 of the suction bell 2 and the seat 12 of the discharge bell 3 can be configured such that the connection is made solely by the protrusion 11 snapping in place in the seat 12 during the interconnection of the suction bell 2 and the discharge bell 3, without performing any further operations.

After that, portions of the torch-shaped member 5 are placed in an opening 16 disposed on the upper portion of the suction bell 2, with the bottom portion of the torch being terminated inside a receiving space 14 of the central member 4 of the suction bell 2. The suction cone 6 is then placed on the top opening of the torch-shaped member 5.

It has to be noted here that the torch-shaped member 5 and the suction cone 6 can also be implemented as an integral member.

This is followed by attaching a connecting element, i.e. a flexible plastic pipe, to the bottom portion 9 of the discharge bell 3, with a connection member known per se, adapted for facilitating the connection of the device 1 to the suction pipe of the vacuum cleaner, being disposed at the other end of the connecting element, situated opposite the discharge bell 3.

The device is now ready for removing a bodily fluid, and can be operated by inserting the suction cone 6 into a nostril of a child, and by removing by suction the bodily fluid applying the vacuum produced by the vacuum cleaner.

The air/bodily fluid mixture flowing at a high velocity - 1.5-2 m/s - is passed via the suction cone 6 and the torch 5 into the receiving space 14 of the central member 4, where the flow direction is reversed, and due to its inertia the fluid is splashed against the wall of the suction bell 2, followed by accumulating at the bottom of the discharge bell 3 while air is carried on unhindered as there is a many thousandfold difference in specific weight between the fluid and air.

Due to the sudden increase of flow diameter, the air flow velocity is significantly reduced - to 1-2 cm/s -, so it cannot carry off the precipitated fluid any more, even if the fluid gets diluted to a near-water consistency.

Under the effect of vacuum, air is carried further along the connection pipe 7 of the discharge bell 3 of the device 1 that is adapted for preventing fluid from being discharged from the device 1 at any spatial orientation thereof.

Due to its configuration, the device 1 can hold approximately 5-6 cm³ of fluid (depending on density) at any given time, which is entirely sufficient for a single instance of removing by suction a bodily fluid.

After use, the device can be disassembled to its components and can be easily cleaned, or, if necessary, sterilized.

The advantage of the device according to the invention is that it can safely remove the fluids accumulated inside the difficult-to-access cavities irrespective of their density. The removed bodily fluids can be safely retained inside the device, completely preventing them from entering the suction device.

### LIST OF REFERENCE NUMERALS

- 1: device
- 2: suction bell
- 3: discharge bell
- 4: central member (sun wheel)
- 5: torch-shaped member
- 6: suction cone
- 7: connection pipe
- 8: upper portion
- 9: bottom portion
- 10: protrusion
- 11: protrusion
- 12: seat
- 13: disc
- 14: receiving space
- 15: bore
- 16: opening

## Claims

1. Device (1) for removing a nasal fluid applying a vacuum source, preferably a vacuum cleaner, comprising a body adapted for receiving a collector vessel fitted with a suction inlet opening and an outlet opening, and a connection pipe (7) configured to be fitted with a connection member for connecting said outlet opening to the vacuum source, wherein said body consists of a suction bell (2) comprising, at one end, an opening (16) receiving a torch-shaped member (5) having a suction cone (6), and, at the other end, a central member (4), and a discharge bell (3) that is connected to the suction bell (2) under the central member (4) and is fitted with said connection pipe (7) at its end situated opposite the central member (4), wherein the central member (4) consists of a receiving space (14) and a disc (13) adapted for bounding the receiving space, with bores (15) being disposed in the surface of the disc (13) and wherein the upper portion (8) of the connection pipe (7) situated in the bottom portion of the discharge bell (3) extends towards the central member (4), the bottom portion (9) of the connection pipe (7) extending over said end of the discharge bell (3).

2. The device (1) according to Claim 2, **characterised in that** the components of the central member (4) are made integrally.

3. The device (1) according to any one of Claims 1 or 3, **characterised in that** the suction bell (2) comprises protrusions (11) at its bottom portion connected to the discharge bell (3), and the discharge bell (3) comprises seats (12) that are disposed at the upper portion thereof and are adapted for receiving the protrusion (11) of the suction bell (2).

4. The device (1) according to Claim 3, **characterised in that** at its bottom free end the discharge bell (3) is fitted with protrusions (10) running parallel with the bottom portion (9) of the connection pipe (7).

5. The device (1) according to any one of Claims 1-4, **characterised in that** the suction bell (2) and the discharge bell (3) have a slightly conical configuration.

6. The device (1) according to any one of Claims 1-5, **characterised in that** the suction bell (2), the discharge bell (3), their subcomponents, and the central member (4), as well as the connector members - such as the torch-shaped member (5) fitted with a suction cone (6) - are made of plastic.

## Patentansprüche

1. Vorrichtung (1) zum Entfernen eines Nasenfluids unter Anlegen einer Vakuumquelle, vorzugsweise eines Staubsaugers, umfassend einen Körper, der zur Aufnahme eines Sammelgefäßes ausgeführt ist, das mit einer Saugeinlassöffnung und einer Auslassöffnung ausgestattet ist, und ein Verbindungsrohr (7), das dazu ausgestaltet ist, mit einem Verbindungsglied zum Verbinden der Auslassöffnung mit der Vakuumquelle ausgestattet zu werden, wobei der Körper aus einer Saugglocke (2) besteht, die an einem Ende eine Öffnung (16), die ein fackelförmiges Glied (5) mit einem Saugkegel (6) aufnimmt, und an dem anderen Ende ein mittleres Glied (4) und eine Austragglocke (3) umfasst, die mit der Saugglocke (2) unter dem mittleren Glied (4) verbunden und an ihrem dem mittleren Glied (4) gegenüberliegenden Ende mit dem Verbindungsrohr (7) ausgestattet ist, wobei das mittlere Glied (4) aus einem Aufnahmeraum (14) und einer zur Begrenzung des Aufnahmeraums ausgeführten Scheibe (13) besteht, wobei Bohrungen (15) in der Oberfläche der Scheibe (13) angeordnet sind und wobei sich der obere Abschnitt (8) des Verbindungsrohrs (7), der in dem unteren Abschnitt der Austragglocke (3) angeordnet ist, zu dem mittleren Glied (4) hin erstreckt, wobei sich der untere Abschnitt (9) des Verbindungsrohrs (7) über das Ende der Austragglocke (3) erstreckt.

2. Vorrichtung (1) nach Anspruch 2, **dadurch gekennzeichnet, dass** die Komponenten des mittleren Glieds (4) integral hergestellt sind.

3. Vorrichtung (1) nach einem der Ansprüche 1 oder 3, **dadurch gekennzeichnet, dass** die Saugglocke (2) an ihrem mit der Austragglocke (3) verbundenen unteren Abschnitt Vorsprünge (11) umfasst und die Austragglocke (3) Sitze (12) umfasst, die an dem oberen Abschnitt davon angeordnet und zur Aufnahme des Vorsprungs (11) der Saugglocke (2) ausgeführt sind.

4. Vorrichtung (1) nach Anspruch 3, **dadurch gekennzeichnet, dass** die Austragglocke (3) an ihrem unteren freien Ende mit parallel zu dem unteren Abschnitt (9) des Verbindungsrohrs (7) verlaufenden Vorsprüngen (10) ausgestattet ist.

5. Vorrichtung (1) nach einem der Ansprüche 1 - 4, **dadurch gekennzeichnet, dass** die Saugglocke (2) und die Austragglocke (3) eine leicht konische Konfiguration aufweisen.

6. Vorrichtung (1) nach einem der Ansprüche 1 - 5, **dadurch gekennzeichnet, dass** die Saugglocke (2), die Austragglocke (3), deren Teilkomponenten und das mittlere Glied (4) sowie die Verbinderglieder wie das mit einem Saugkegel (6) ausgestattete fackelförmige Glied (5) aus Kunststoff hergestellt sind.

## Revendications

1. Dispositif (1) d'évacuation d'un fluide nasal appliquant une source de vide, de préférence un aspirateur, comprenant un corps adapté à recevoir un récipient collecteur muni d'une ouverture d'entrée d'aspiration et d'une ouverture de sortie, et un tuyau de raccordement (7) configuré pour être muni d'un organe de raccordement pour raccorder ladite ouverture de sortie à la source de vide, ledit corps étant constitué d'une cloche d'aspiration (2) comprenant, à une extrémité, une ouverture (16) recevant un organe en forme de torche (5) présentant un cône d'aspiration (6), et à l'autre extrémité, un organe central (4), et une cloche d'évacuation (3) raccordée à la cloche d'aspiration (2) sous l'organe central (4) et équipée dudit tuyau de raccordement (7) à son extrémité située à l'opposé de l'organe central (4), l'organe central (4) étant constitué d'un espace de réception (14) et d'un disque (13) adapté pour délimiter l'espace de réception, avec des alésages (15) qui sont disposés dans la surface du disque (13), et la partie supérieure (8) du tuyau de raccordement (7) située dans la partie inférieure de la cloche d'évacuation (3) s'étendant vers l'organe central (4), la partie inférieure (9) du tuyau de raccordement (7) s'étendant sur ladite extrémité de la cloche d'évacuation (3).

2. Dispositif (1) selon la revendication 2, **caractérisé en ce que** les composants de l'organe central (4) sont réalisés d'une seule pièce.

3. Dispositif (1) selon l'une quelconque des revendications 1 ou 3, **caractérisé en ce que** la cloche d'aspiration (2) comprend des saillies (11) à sa partie inférieure raccordée à la cloche d'évacuation (3), et la cloche d'évacuation (3) comprend des sièges (12) qui sont disposés à sa partie supérieure et sont adaptés pour recevoir la saillie (11) de la cloche d'aspiration (2).

4. Dispositif (1) selon la revendication 3, **caractérisé en ce que** la cloche d'évacuation (3) est munie, à son extrémité inférieure libre, de saillies (10) s'étendant parallèlement à la partie inférieure (9) du tuyau de raccordement (7).

5. Dispositif (1) selon l'une quelconque des revendications 1 à 4, **caractérisé en ce que** la cloche d'aspiration (2) et la cloche d'évacuation (3) ont une configuration légèrement conique.

6. Dispositif (1) selon l'une quelconque des revendications 1 à 5, **caractérisé en ce que** la cloche d'aspiration (2), la cloche d'évacuation (3), leurs sous-composants et l'organe central (4), ainsi que les éléments de raccordement - tels que l'organe en forme de torche (5) équipé d'un cône d'aspiration (6) - sont en matière plastique.
